# EUROPEAN PATENT APPLICATION

(11) **EP 4 725 514 A2**
(43) Date of publication of application: **15.04.2026**
(21) Application number: 25210999.6
(22) Date of filing: 21.12.2020
(51) Int. Cl.: A61L 27/38

(54) **COMPOSITION FOR IMPROVED BONE FRACTURE HEALING**

(30) Priority: 24.12.2019 GB 201919285
(62) Divisional of application: 20838248.1
(71) Applicant: Corthotec Ltd, London E1 0SG (GB)
(72) Inventor: KALLALA, Rami, GREATER LONDON, SM6 7ER (GB)
(74) Representative: Eisenführ Speiser

(57) **Abstract**

The present invention relates to a composition for use as an adjunct in orthopaedic surgery, such as in the treatment of i) delayed fracture healing in bone, manifesting as either a delayed or non-union; ii) in a fusion procedure anywhere in the skeletal system, such as cranial, spinal, foot and ankle, or upper limb; and iii) for use as a bone void filler and to enhance bone in-filling in situations of bone loss such as following combat, e.g. blast injuries, or non-combat related trauma such as roadtraffic accidents.

## Description

The present invention relates to a composition for use as an adjunct in orthopaedic surgery, such as in the treatment of delayed fracture healing in bone, or in a fusion procedure anywhere in the skeletal system.

The total economic impact of musculoskeletal conditions represents $126 billion in the US alone, with approximately 6.8 million fractures requiring orthopaedic input annually. Of these 6.8 million fractures, approximately 300,000 per year will go on to develop slow (delayed) or incomplete (non-union) healing. Non-union is a term generally used to refer to a fracture that has failed to heal within 9 months. In the UK, there are approximately 850,000 new fractures seen each year, of which the majority heals without any significant complication. Rates of non-union of 5-10% of fractures have been suggested. A proportion of these will be accounted for by fragility fractures due to poor bone density in elderly patients, as with an increasing global population and aging society, an ever-increasing number of fragility fractures occur worldwide. An estimated 200 million people worldwide suffer from osteoporosis, of which 40% of female sufferers and 15-30% of men will sustain one or more fragility fractures in their remaining lifetime. Typically, these patients demonstrate impaired fracture healing, with consequent increases in operative intervention, increased risk of complications and incidence of delayed and non-union healing. In addition, certain fractures are known for their poor healing potential. For example, scaphoid fractures carry an associated risk of non-union approaching 50%. As well as the fracture type (location, blood supply, open/closed), patient factors also determine fracture-healing potential, with, for example, diabetic patients, or those on long-term steroid and immunosuppressant therapy also demonstrating impaired fracture healing.

Delayed fracture healing and non-union present a particularly challenging problem for care providers and patients, and therefore the health system and social services supporting them. Their management typically requires large assets and long-lasting therapies with often unsuccessful results.

Several studies have analysed the health economics of non-union, all demonstrating major increases in length of hospital stays, number of operations, and the lengths of follow up treatments. A 2007 paper by Kanakaris et al found that on average the cost of treatment for non-union ranged from £15,660 to £17,200, dependent upon the location of the fracture and treatment modality used. A separate paper by Dahabreh *et al* analyzed 25 fractures with non-union, and their economic analysis focused on the direct medical costs of in-hospital and outpatient treatments from the time of initial injury. A total of 127 hospital admissions, a mean hospital stay of 34.08 days and a mean of 5.36 operations per fracture were recorded. The average total cost of treatment per fracture that develops a non-union was found to be £21,183.05. This was in contrast to the same fractures if treated successfully, with an uncomplicated clinical course, costing from £3,003 to £3,119. Patil *et al* published a paper focusing on tibial and femoral non-unions treated with an Ilizarov frame. The mean number of operations undergone prior to the surgical intervention of the authors was 3. The estimated costs were derived from the 2004-2005 assessment of the Finance Department of their hospital, and were limited to the final phase of treating these 41 complicated cases with an Ilizarov frame. The average expenses reached the sum of £29,204 and represented the direct medical costs of this treatment option alone.

The cost to the UK National Health Service (NHS) of treating non-union has been reported to range between £7,000 and £79,000 per person. However, this does not take into account the morbidity and loss of earnings of the individual, nor any long-term health burden, so the cost to society will in fact be far greater than this. The overall burden in the UK on the National Health Service is therefore significant. Extrapolating up from an estimated 85,000 non-unions per annum in the UK would give a financial cost of £595 million to £6.7 billion based on available data, notwithstanding the patient burden of pain, associated depression, disability and time off work, with the resulting negative impact upon productivity and therefore economic impact on the nation as a whole.

The majority of health systems in Europe are tax-based government funded systems. As a result, a reduction in this burden by up to 50% would provide a significant health budget saving and improvement in quality of life. A best-case scenario of a reduction in incidence of 50% would deliver savings of £297.5 million to £3.6 billion per year.

Delayed and non-union fractures are typically managed with a combination of surgery and biological methods. Surgery involves either debridement of the non-union site and internal fixation such as plate fixation or over-reaming and intramedullary nailing, or external fixation systems such as Ilizarov circular frames. Biological methods revolve around filling osseous defects and encouraging the fracture repair process, either in the form of autologous bone graft, or bone graft substitutes - both allogenic or synthetic. Platelet rich plasma and bone marrow aspirate are also used in combination with a bone graft to enhance fracture healing, but are harvested from the patient, or donors, usually sourced from national tissue banks in the UK rather than commercial sources. In addition, orthobiologics are also starting to be used; chiefly the bone morphogenetic proteins (BMP) 2 and 7, with several studies demonstrating their ability to create ectopic bone formation by recruiting stem cells from distant sites and inducing osteoblast and chondrocyte differentiation at the fracture site. However, they have failed to become as pervasive as anticipated due to their relatively high cost, with one vial of BMP-7 costing £3000. Despite this, the benefit of orthobiologics in the treatment of fracture non-unions is emerging, with one study reporting a cost saving of 47% when treating non-unions with BMP-7 compared to traditional treatment methods alone.

It would therefore be desirable to develop a composition which is able to aid in the treatment of chronic bone injuries and fractures that have developed a delayed or non-union, or which are able to be used in a fusion procedure anywhere in the skeletal system, and which are able to reduce the incidence of surgical methods in the treatment thereof. Ideally, such a composition would also be able to shorten the duration of fracture healing.

Therefore, in accordance with the invention, there is provided a composition comprising:
i) a parathyroid hormone or a derivative thereof in an amount of approximately 0.1 ng/ml to approximately 50 ng/ml;
ii) one or more osteoclast inhibitors; and
iii) a bone void filler in a solid or liquid phase;

wherein the bone void filler is selected from calcium sulphate, or mono-, di-, and tri-calcium phosphates, calcium carbonate, an autograft bone material, an allograft, a synthetic allograft, a ceramic, a bioglass, a collagen sponge, carboxymethylcellulose or a polymethyl methacrylate (PMMA) bone cement, or a or combination of any two or more thereof; and
wherein the one or more osteoclast inhibitors are present in an amount of approximately 1 ng/ml to approximately 6000 ng/ml.

In one embodiment of the invention, the osteoclast inhibitor comprises a bisphosphonate or a derivative thereof; strontium ranelate, denosumab, or romosozumab; or a combination of any two or more thereof.

By 'bone void filler' is meant herein a bioinert physiologically acceptable material which is to be introduced into a cavity, hole or void in a human or animal bone, which may for example have been caused by disease or trauma, and provides an osteoconductive matrix to facilitate the repair of the bone. The bone void filler will also gradually resorb into the body or dissolve over time.

The composition of the invention is intended to be used intra-operatively, that is, to be prepared and utilised in a sterile operating room during a surgical procedure, for implantation into an indicated patient in order to effect treatment, as an adjunct to surgical care.

The composition of the invention is intended to be for use as an adjunct in orthopaedic surgery. Exemplary uses for the composition include, but are not limited to, in the treatment of (i) delayed fracture healing in bone, manifesting as either a delayed or non-union, whether due to fracture-specific factors (*i.e.* the location of the fracture; or whether it is open or closed), or patient-specific factors; (ii) in a fusion procedure anywhere in the skeletal system, such as cranial, spinal, foot and ankle, or upper limb; and (iii) for use as a bone void filler and to enhance bone in-filling in situations of bone loss such as following combat, such as blast injuries or non-combat related trauma, e.g. road-traffic accidents.

According to one embodiment of the invention, the composition comprises the parathyroid hormone or a derivative thereof (denoted herein as PTH), the one or more osteoclast inhibitors - such as a bisphosphonate or a derivative thereof; or other osteoclast inhibitors such as strontium ranelate, denosumab, or romosozumab; or a combination of any two or more thereof, and the bone void filler as independent entities. In this embodiment, chemically the PTH and one or more osteoclast inhibitors are independent molecules sitting in a matrix of the bone void filler.

According to another embodiment of the invention, the composition comprises the PTH in a conjugated form with the one or more osteoclast inhibitors, in combination with the bone void filler. In all other respects, these two embodiments are the same.

In one embodiment of the invention, the composition comprises a bone void filler, such as a calcium sulphate-based bone void filler, with a primary mechanism of action (PMOA) that is enhanced by the addition of actives. This results in a change of PMOA from solely osteoconduction with calcium sulphate, to include osteoinduction and osteogenesis with the addition of the actives. These actives comprise parathyroid hormone or a derivative thereof in an amount of approximately 0.1 ng/ml to approximately 50 ng/ml; and one or more osteoclast inhibitors, such as a bisphosphonate or a derivative thereof; strontium ranelate, denosumab, or romosozumab; or a combination of any two or more thereof.

In this form the PMOA of the device is achieved by calcium sulphate acting as a bone scaffold allowing conduction of bone cells in a bone void to encourage bone in-filling. If for example PTH in any form, with an osteoclast inhibitor in any form, were added to the calcium sulphate, then in addition to acting as a scaffold, the action of the PMOA would be *enhanced* to cause the medical device to have the additional two properties of osteoinduction and osteogenesis described above, encouraging new bone formation.

The parathyroid hormone, or a derivative thereof, used in the composition of the invention may be produced in, or derived from, any form, such as, for example, by either recombinant means or synthetically.

The use of the PTH acts two-fold in the composition of the invention; firstly, as a stem cell recruiter (such as for mesenchymal stem cells), increasing the number of cell substrates for use in the fracture repair process; and secondly, in increasing the longevity of osteoblasts involved in the fracture repair process. These effects have been demonstrated in animals exposed to high systemic doses of PTH, with emerging evidence in humans; however few studies to date have used topically applied PTH, and none have applied PTH via the preferred delivery method of the invention, which is discussed further below.

Examples of derivatives of parathyroid hormone which may be used in the composition of the invention include, but are not limited to, parathyroid hormone-related protein (PTHrP), 1-34 recombinant/synthesised human parathyroid hormone (1-34 rhPTH), 1-84 recombinant/synthesised human parathyroid hormone (1-84 rhPTH), H05AA03 parathyroid hormone, teriparatide acetate, preotact parathyroid hormone, teriparatide, abaloparatide, or combinations of any two or more thereof.

The parathyroid hormone or derivative thereof is present in an amount of 0.1 ng/ml to approximately 50 ng/ml; more typically between approximately 0.25 ng/ml to approximately 40 ng/ml; more typically between approximately 0.5 ng/ml to approximately 30 ng/ml; more typically between approximately 1 ng/ml to approximately 20 ng/ml. In another embodiment, the amount of the parathyroid hormone or derivative thereof may be from approximately 2 ng/ml to approximately 18 ng/ml, or approximately 5 ng/ml to approximately 15 ng/ml.

In summary, the composition of the invention acts in two ways. PTH increases the number of cells available at the fracture site for the fracture repair process; and the one or more osteoclast inhibitors, such as a bisphosphonate (or a derivative thereof; or strontium ranelate, denosumab, or romosozumab; or a combination of any two or more thereof), prevents unwanted osteoclast activity at this stage.

PTH-based therapy has been observed to have complex effects on bone healing, depending upon its particular route of administration, its combination with other agents and the interval of delivery.

The present inventors have shown that the PTH cell response is dose dependent and that therapeutic efficacy can be enhanced in combination with an osteoclast inhibitor, such as zoledronic acid, within a sustained release formulation designed fully to elute its active components within a maximum of a 6-week period, in order to maximise clinical efficacy and promote rapid healing.

Compelling feasibility studies (both *in vitro* and *in vivo*) indicate that the present invention promotes osteoblast viability and has the potential to promote fracture healing and prevent non-union from occurring in both acute and chronic fractures.

Additionally, it has been demonstrated by the present inventors that the invention has the ability to improve clinical outcomes in patients with acute fractures at high risk of non-union whether due to fracture-specific factors (location, open/closed) or patient-specific factors and chronic injuries that have developed a delayed or non-union bone fracture.

When the one or more osteoclast inhibitors comprises a bisphosphonate, one or more bisphosphonates may be present in the composition of the invention. Examples of bisphosphonates suitable for use in compositions of the invention include, but are not limited to, zoledronic acid, alendronic acid, an etidronate (such as the disodium salt), pamidronate (such as the disodium salt), ibandronic acid, a risedronate (such as the disodium salt), and/or a clodronate (such as the disodium salt), or a combination of any two or more thereof. Also, compounds such as strontium ranelate and/or denosumab (available under the trade names Prolia^{®} and Xgeva^{®}), or romosozumab (available under the trade name Evenity^{®}), could be used alone or in combination with each other, or with any of the bisphosphonates named above.

When the bisphosphonate comprises zoledronic acid, alendronic acid or ibandronic acid, they are typically present in an amount of from approximately 50 ng/ml to approximately 300 ng/ml, more typically from approximately 75 ng/ml to approximately 250 ng/ml, still more typically from approximately 100 ng/ml to approximately 200 ng/ml.

When the bisphosphonate comprises an etidronate, it is typically present in an amount of from approximately 300 ng/ml to approximately 1500 ng/ml, more typically from approximately 500 ng/ml to approximately 1250 ng/ml, still more typically from approximately 750 ng/ml to approximately 1200 ng/ml, still more typically from approximately 900 ng/ml to approximately 1100 ng/ml.

When the bisphosphonate comprises a pamidronate, it is typically present in an amount of from approximately 0.01 nmol/ml to approximately 20 nmol/ml, more typically from approximately 1 nmol/ml to approximately 18 nmol/ml, still more typically from approximately 5 nmol/ml to approximately 15 nmol/ml, still more typically from approximately 8 nmol/ml to approximately 12 nmol/ml.

When the bisphosphonate comprises a risedronate, it is typically present in an amount of from approximately 0.01 ng/ml to approximately 50 ng/ml, more typically from approximately 1 ng/ml to approximately 40 ng/ml, still more typically from approximately 2 ng/ml to approximately 30 ng/ml, still more typically from approximately 5 ng/ml to approximately 25 ng/ml, still more typically from approximately 6 ng/ml to approximately 20 ng/ml, still more typically from approximately 7 ng/ml to approximately 15 ng/ml, still more typically from approximately 8 ng/ml to approximately 12 ng/ml.

When the bisphosphonate comprises a clodronate, it is typically present in an amount of from approximately 500 ng/ml to approximately 3000 ng/ml, more typically from approximately 550 ng/ml to approximately 2800 ng/ml, still more typically from approximately 600 ng/ml to approximately 2700 ng/ml, still more typically from approximately 700 ng/ml to approximately 2600 ng/ml.

In one embodiment of the invention, the bisphosphonate comprises zoledronic acid, or is zoledronic acid.

Also envisaged within the invention is that the composition may further comprise one or more additives selected from vitamin D and its derivatives or isomers thereof, hydroxyapatite and its derivatives or isomers thereof, vitamin E and its derivatives or isomers thereof, selenium, zinc, magnesium, phosphate, or collagen. Examples of derivatives of vitamin D include 1,25(OH)₂ vitamin D3 and its synthetic derivatives 1,24(OH)₂ vitamin D3, calcitonin, and calcipotriol.

According to a further aspect of the invention, the composition may further be combined with stem cells, such as mesenchymal stem cells.

According to one embodiment of the invention, the bone void filler comprises calcium sulphate, such as a hemihydrate of calcium sulphate.

The composition of the invention is either employed in a form suitable for injection into a body, or in the form of solid set pellets for implantation into the body.

In one form, the composition is a paste which arrives unmixed in its individual constituent parts, and is then mixed together intraoperatively in a theatre operating room. While still in paste form, it is then spread across a rubber mould to create pellets. This paste usually sets in approximately 5-10 mins, depending on what is mixed into it (*i.e.* size/complexity of solutes). The pellets sizes are typically approximately 2 mm diameter (for use in an upper limb), or approximately 5 mm diameter (for use in a lower limb).

The composition of the invention may be administered to an affected area of the patient by any method apparent to a medical professional. However, according to one embodiment of the invention, the composition is mixed with the bone void filler, such as a calcium sulphate paste, for direct injection into a fracture site. In acute fractures, a percutaneous technique under fluoroscopic guidance could be used, whereas in a chronic injury, a small incision and osteotomy or drilling to access the fracture site may be needed. Once injected into a bony space its setting time is approximately 8 minutes, followed by a complete absorption time of 2-3 weeks, over which it elutes any added compounds. After this period, the product is absorbed entirely, leaving no trace either clinically or radiographically.

However, any other bone void filler defined hereinabove may also be used to deliver the composition to the body of a subject.

According to a further aspect of the invention, there is provided a kit of parts for the manufacture of a composition as defined hereinabove, the kit of parts comprising:
i) a parathyroid hormone or a derivative thereof;
ii) one or more osteoclast inhibitors; and
iii) a bone void filler selected from calcium sulphate, or mono-, di-, and tri-calcium phosphates, calcium carbonate, an autograft bone material, an allograft, a synthetic allograft, a ceramic, a bioglass, a collagen sponge, carboxymethylcellulose or a polymethyl methacrylate (PMMA) bone cement, or a or combination of any two or more thereof.

The kit may comprise the bone void filler, such as calcium sulphate powder, PTH (which is typically in solution), and one or more osteoclast inhibitors, such as a bisphosphonate (or a derivative thereof; or strontium ranelate, denosumab, or romosozumab; or a combination of any two or more thereof), which is also typically in solution. All three components are typically mixed together with an additional small aliquot of sterile water into a fast setting paste, which can be either injected in a liquid form into a bony defect where it sets solid, or spread across a sterile custom rubber mould included in the kits, which the paste sets on and produces solid pellets.

According to a further aspect of the invention, there is provided a method of manufacturing a composition as herein described, the method comprising:
i) a parathyroid hormone or a derivative thereof in an amount of approximately 0.1 ng/ml to approximately 50 ng/ml; and
ii) one or more osteoclast inhibitors; and
iii) a bone void filler for delivery of the composition in a solid or liquid phase to a subject;

wherein the bone void filler is selected from calcium sulphate, or mono-, di-, and tri-calcium phosphates, calcium carbonate, an autograft bone material, an allograft, a synthetic allograft, a ceramic, a bioglass, a collagen sponge, carboxymethylcellulose or a polymethyl methacrylate (PMMA) bone cement, or a or combination of any two or more thereof; and wherein one or more osteoclast inhibitors are present in an amount of approximately 1 ng/ml to approximately 6000 ng/ml;
the method comprising combining the parathyroid hormone or derivative thereof with the one or more osteoclast inhibitors, and an amount of the bone void filler.

According to a further aspect of the invention, there is provided a composition as defined hereinabove, in the treatment of bone fractures or in a bone fusion procedure. The treatment typically involves administering the composition to a patient either by injection in a liquid form into a bony defect where it sets solid, or as one or more solid pellets.

According to a further aspect of the invention, there is provided a method of delivering a composition as defined herein above into a human or animal body.

The present invention will also be further explained with reference to the following figures:

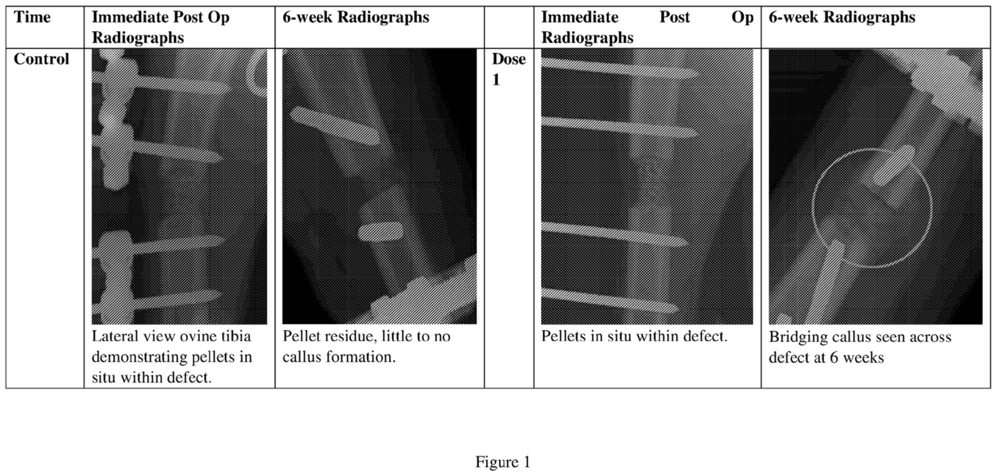

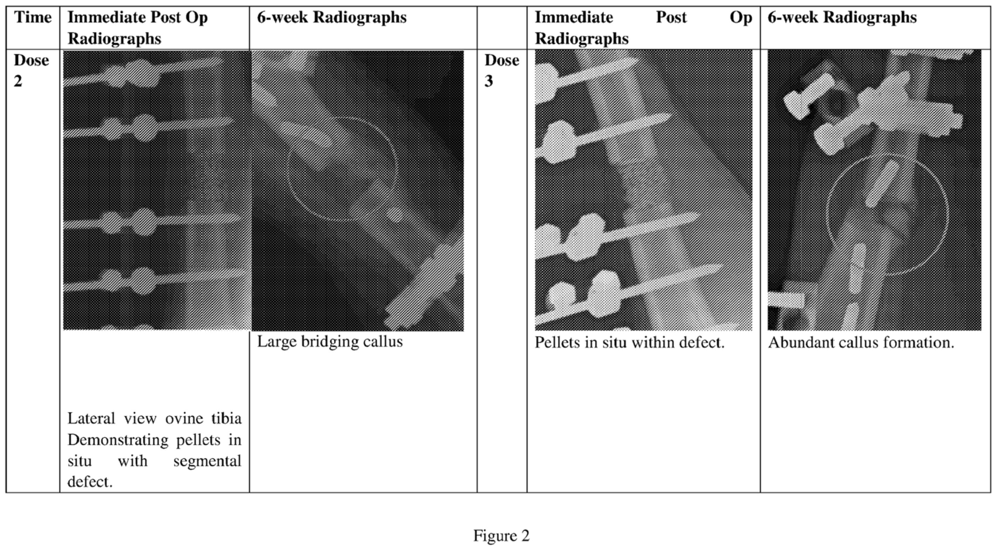

Figures 1 and 2 refer to an ovine tibial critical segmental defect model of non-union that was used to demonstrate efficacy of the invention in three doses within the therapeutic range of PTH 0.1 ng/ml to approximately 50 ng/ml, and an amount of osteoclast inhibitor that is within the therapeutic range of 1 ng/ml to approximately 6000 ng/ml. This is an established large-animal model of non-union (Reichert et al, TISSUE ENGINEERING: Part B Volume 00, Number 00, 2009*).*
Across Doses 1-3, a range of different concentrations of the components have been used. These doses correspond to each other in the ratio of 1:2:5 and have been used to demonstrate the composition according to the invention, containing an amount of parathyroid hormone that is within the therapeutic range of 0.1 ng/ml to 50 ng/ml, and an amount of osteoclast inhibitor that is within the therapeutic range of 1 ng/ml to approximately 6000 ng/ml. In this study, the bisphosphonate selected in the composition was zoledronic acid combined with 1-34 teriparatide. The control used was plain calcium sulphate pellets.
Figure 1 shows the immediate post-operation radiographs and the 6-week radiographs taken of an ovine tibia with treatment using a control and a composition according to the invention with an amount of parathyroid hormone within the therapeutic range of 0.1 ng/ml to approximately 50 ng/ml, and an amount of osteoclast inhibitor that is within the therapeutic range of 1 ng/ml to approximately 6000 ng/ml (Dose 1).
Figure 2 shows the immediate post-operation radiographs and the 6-week radiographs taken of an ovine tibia with treatment using two different compositions according to the invention with an amount of parathyroid hormone within the therapeutic range of 0.1 ng/ml to approximately 50 ng/ml, and an amount of osteoclast inhibitor that is within the therapeutic range of 1 ng/ml to approximately 6000 ng/ml (Doses 2 and 3).
Figure 3 shows a graph depicting the optimal concentrations of zoledronate required in respect of bone resorption.
Figure 4 shows a graph depicting the optimal concentrations of zoledronate required in respect of osteoclast number.
Figure 5 shows graphs depicting the elution/release profile of a composition according to the invention in terms of the amount of the zoledronic acid and rhPTH(1-34) polypeptide released over time.
Figures 6-8 show axial CT scans of an ovine tibia using a control and three different compositions according to the invention, taken at 3, 6 and 12 weeks after treatment was commenced.

In Figure 1, a control containing only calcium sulphate was used to treat an ovine model of non-union. In the immediate post-operation radiograph, the presence of the pellets of the composition of the invention can be seen in the bone defect.

However, in the radiograph taken 6 weeks after the operation, the partially resorbed pellets can still be seen and there is little callus present. Callus is the bulge of new immature bone formation seen in X-rays and is a crucial stage in bone healing. The lack of callus formation indicates repair of the bone defect has not occurred and that the subsequent recovery of the patient will be delayed.

On the right-hand side of Figure 1 is the same situation, but this time using a composition according to the invention (Dose 1). Dose 1 corresponds to a composition according to the invention, containing an amount of parathyroid hormone that is within the therapeutic range of 0.1 ng/ml to 50 ng/ml, and an amount of osteoclast inhibitor that is within the therapeutic range of 1 ng/ml to approximately 6000 ng/ml. Again, in the immediate post-operation radiograph, the presence of the pellets of the composition of the invention can be seen in the bone defect.

However, in the radiograph taken 6 weeks after the operation, a bridging callus can clearly be seen across the bone defect. This shows that the bone defect, *e.g.* the critical segmental defect, is being filled with new bone, and at a significantly faster rate than the calcium sulphate control.

In Figure 2, the same is demonstrated. Both of the examples in Figures 2 employed compositions according to the invention (Doses 2 and 3). Across Doses 1-3, a range of different concentrations of the PTH have been used, and these doses correspond to each other in the ratio of 1:2:5. Doses 2 and 3 corresponded to each other in a ratio of 2:5 in terms of composition according to the invention, containing an amount of parathyroid hormone that is within the therapeutic range of 0.1 ng/ml to 50 ng/ml, and an amount of osteoclast inhibitor that is within the therapeutic range of 1 ng/ml to approximately 6000 ng/ml. Again, in the immediate post-operation radiograph, the presence of the pellets of the composition of the invention can be seen in these bone defects.

However, in the radiographs taken 6 weeks after the operation, large bridging calluses can clearly be seen across the bone defect. As with Dose 1 In Figure 1, this shows that the critical segmental defect is being filled with new bone, and at a significantly faster rate than the calcium sulphate control.

These X-rays therefore demonstrate a significantly superior bone callus formation at 6 weeks with the compositions according to the invention, compared with a control containing only calcium sulphate. The presence of the callus in the doses employing compositions according to the invention is critical in the recovery of a patient to a non-union bone fracture.

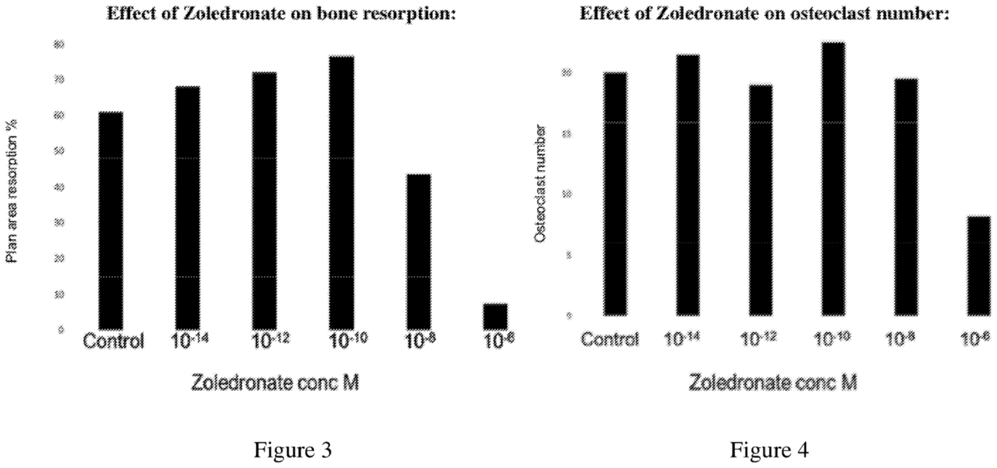

The graphs in Figure 3 and 4 depict the optimal concentrations of zoledronate (derived from zoledronic acid as the bisphosphonate) required to supress bone resorption by means of a proportionate decrease in osteoclast number.

Figures 3 and 4 demonstrate optimal efficacy of zoledronic acid at a concentration of approximately 10⁻⁶M of zoledronic acid, at which concentration it can be seen that the amount of osteoclast activity (as measured by bone resorption) decreases significantly in correlation with decreasing osteoclast number, when compared to lower concentrations of zoledronic acid.

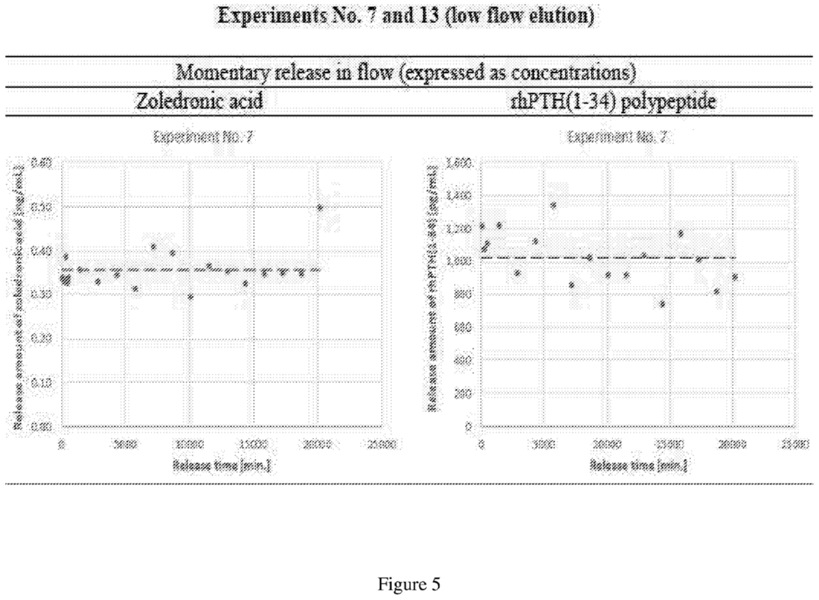

The elution profiles illustrated in Figure 5 demonstrate the composition according to the invention is able to achieve a controlled release over a period of time, to facilitate the bone repair process. 20000 minutes equates to 47.6 days, or just under 7 weeks.

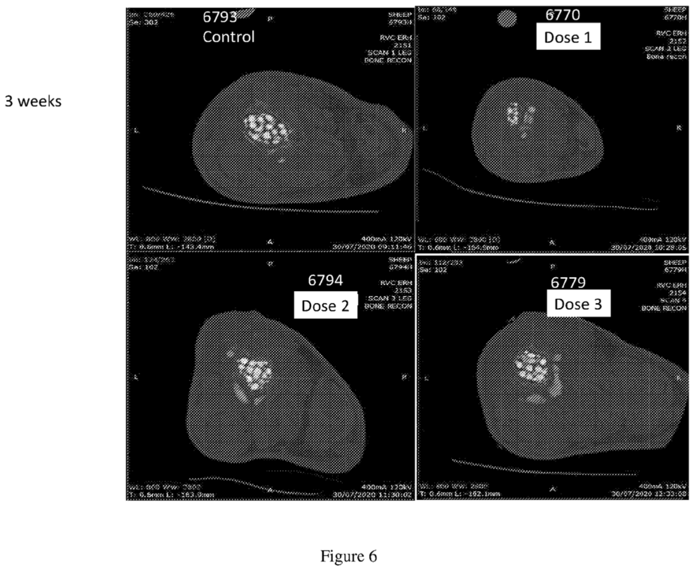

In Figures 6-8, the respective CT scans of an ovine tibia using a control and three different compositions according to the invention, taken at 3, 6 and 12 weeks after treatment was commenced, are shown. Again, the compositions according to the invention contain a range of different concentrations of the PTH, corresponding to each other in the ratio of 1:2:5, while the control contains calcium sulphate only.

In Figure 6, the axial CT scan demonstrates pellets in situ with early callus formation at 3 weeks, versus the control. The amount of callus formed is incremental with increasing dose, from Dose 1 to Dose 3.

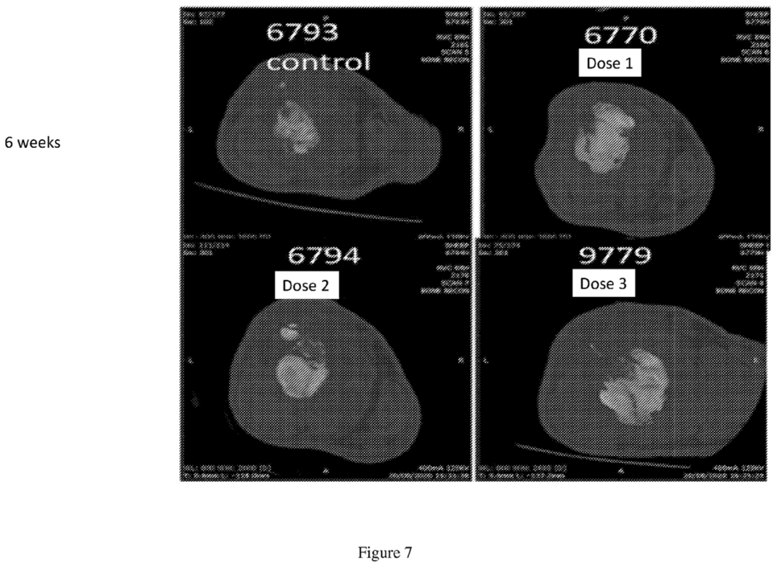

In Figure 7, the axial CT scan demonstrates abundant callus formation at 6 weeks of treatment, versus the control. The amount of callus formed is incremental with increasing dose, from Dose 1 to Dose 3.

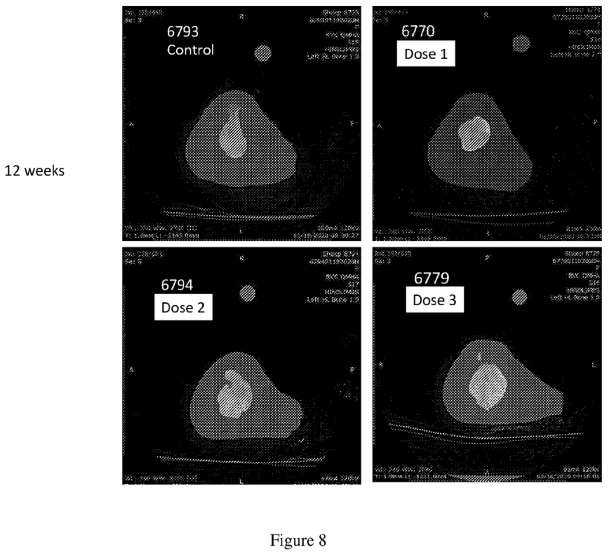

In Figure 8, the axial CT scan demonstrates greater bone volume at 12 weeks of treatment, versus the control. The amount of new bone formation is incremental with increasing dose, from Dose 1 to Dose 3.

This study indicates that PTH has a positive effect on fracture healing, and qualitative assessment indicated that bone formation was greater in the animals treated with PTH compared with the control. This was particularly true at the early time points. Longitudinal assessment using CTs and radiographs is important because 12-week evaluation disguises the positive effects that PTH may have on early fracture healing. In all the three animals treated with PTH there is evidence of bone bridging the osteotomy gap earlier than with the controls. This is important because early fracture healing may lead to reduced non-unions or reduced delayed fracture union.

A comprehensive study of the use of calcium sulphate-based bone substitutes in revision lower limb arthroplasty reported an average reabsorption period of 6 to 8 weeks (Kallala et al; Bone Joint Res, 2018; 7:570-579*;* McPherson et al; Dissolvable Antibiotic Beads in Treatment of Periprosthetic Joint Infection and Revision Arthroplasty - The Use of Synthetic Pure Calcium Sulfate (Stimulan®) Impregnated with Vancomycin & Tobramycin. Reconstr Rev 2013;3:32-43*).*

The present invention has therefore demonstrated that the composition according to the invention, containing an amount of parathyroid hormone that is within the therapeutic range of 0.1 ng/ml to 50 ng/ml, and an amount of osteoclast inhibitor that is within the therapeutic range of 1 ng/ml to approximately 6000 ng/ml, is also able to achieve this, while simultaneously also demonstrating superior bone callus formation at 6 weeks, to facilitate bone healing. Alternative materials, such as calcium phosphate or hydroxyapatites, result in prolonged reabsorption over months, not weeks.

Therefore, it can clearly be seen that for the present invention, the PMOA of the bone void filler is enhanced by the addition of the PTH or a derivative thereof in an amount of approximately 0.1 ng/ml to approximately 50 ng/ml, and one or more osteoclast inhibitors. The PMOA is changed from solely osteoconduction with the bone void filler alone, also to include osteoinduction and osteogenesis with the addition of the other components.

There is a synergistic effect between the bone void filler and the PTH and one or more osteoclast inhibitors, which enhances the PMOA for the bone void filler.

It is of course to be understood that the present invention is not intended to be restricted to the foregoing examples which are described by way of example only.

The invention further comprises the following embodiments:
1. A composition comprising:
   i) a parathyroid hormone or a derivative thereof in an amount of approximately 0.1 ng/ml to approximately 50 ng/ml;
   ii) one or more osteoclast inhibitors; and
   iii) a bone void filler in a solid or liquid phase;

   wherein the bone void filler is selected from calcium sulphate, or mono-, di-, and tri-calcium phosphates, calcium carbonate, an autograft bone material, an allograft, a synthetic allograft, a ceramic, a bioglass, a collagen sponge, carboxymethylcellulose or a polymethyl methacrylate (PMMA) bone cement, or a or combination of any two or more thereof; and
   wherein the one or more osteoclast inhibitors are present in an amount of approximately 1 ng/ml to approximately 6000 ng/ml.
2. A composition according to embodiment 1, wherein the one or more osteoclast inhibitors comprise a bisphosphonate or a derivative thereof; strontium ranelate, denosumab, or romosozumab; or a combination of any two or more thereof.
3. A composition according to embodiment 2, wherein the bisphosphonate is selected from zoledronic acid, alendronic acid, an etidronate, a pamidronate, ibandronic acid, a risedronate, and/or a clodronate, or a combination of any two or more thereof.
4. A composition according to embodiment 3, wherein when the bisphosphonate comprises zoledronic acid, alendronic acid or ibandronic acid, they are present in an amount of from approximately 50 ng/ml to approximately 300 ng/ml; when the bisphosphonate comprises an etidronate, it is present in an amount of from approximately 300 ng/ml to approximately 1500 ng/ml; when the bisphosphonate comprises a pamidronate, it is typically present in an amount of from approximately 0.01 nmol/ml to approximately 20 nmol/ml; when the bisphosphonate comprises a risedronate, it is typically present in an amount of from approximately 0.01 ng/ml to approximately 50 ng/ml; and/or when the bisphosphonate comprises a clodronate, it is typically present in an amount of from approximately 500 ng/ml to approximately 3000 ng/ml.
5. A composition according to any preceding embodiment, wherein the bisphosphonate comprises, or is, zoledronic acid.
6. A composition according to any preceding embodiment, wherein the derivative of the parathyroid hormone is selected from parathyroid hormone-related protein (PTHrP), 1-34 recombinant human parathyroid hormone (1-34 rhPTH), 1-84 recombinant human parathyroid hormone (1-84 rhPTH), H05AA03 parathyroid hormone, preotact parathyroid hormone, teriparatide, abaloparatide, or combinations of any two or more thereof.
7. A composition according to any preceding embodiment, further comprising one or more additives selected from vitamin D and its derivatives or isomers thereof, hydroxyapatite and derivatives or isomers thereof, vitamin E and derivatives or isomers thereof, selenium, zinc, magnesium, phosphate, or collagen and derivatives or isomers thereof.
8. A composition according to any preceding embodiment, further comprising stem cells.
9. A composition according to embodiment 8, wherein the stem cells are mesenchymal stem cells.
10. A composition according to any preceding embodiment, wherein the composition is in a solid form.
11. A composition according to any of embodiments 1-9, wherein the composition is in a liquid form.
12. A method of manufacturing a composition according to any preceding embodiment, the method comprising combining (i) the parathyroid hormone or derivative thereof; (ii) the one or more osteoclast inhibitors; and (iii) the bone void filler.
13. A composition according to any of embodiments 1-11 in the treatment of bone fractures, or in a bone fusion procedure.
14. A method of delivering a composition according to any of embodiments 1-11 into a human or animal body.
15. A method according to embodiment 14, wherein the composition is delivered via injection or in the form of solid pellets.
16. A kit of parts for the manufacture of a composition according to any of embodiments 1-11, the kit of parts comprising:
   i) a parathyroid hormone or a derivative thereof;
   ii) one or more osteoclast inhibitors; and
   iii) a bone void filler selected from calcium sulphate, or mono-, di-, and tri-calcium phosphates, calcium carbonate, an autograft bone material, an allograft, a synthetic allograft, a ceramic, a bioglass, a collagen sponge, carboxymethylcellulose or a polymethyl methacrylate (PMMA) bone cement, or a or combination of any two or more thereof.

## Claims

1. A composition comprising:
(i) a parathyroid hormone or a derivative thereof in an amount of approximately 0.1 ng/ml to approximately 50 ng/ml;
(ii) one or more osteoclast inhibitors; and
(iii) a bone void filler in a solid or liquid phase;
wherein the one or more osteoclast inhibitors comprise a bisphosphonate.

2. The composition according to claim 1, wherein the one or more osteoclast inhibitors further comprise strontium ranelate, denosumab, or romosozumab; or a combination of any two or more thereof; and/or
wherein the bisphosphonate is selected from zoledronic acid, alendronic acid, an etidronate, a pamidronate, ibandronic acid, a risedronate, and a clodronate, or a combination of any two or more thereof.

3. The composition according to claim 1 or claim 2, wherein the bisphosphonate comprises zoledronic acid, alendronic acid, or ibandronic acid.

4. The composition according to any one of claims 1 to 3, wherein the osteoclast inhibitor is in an amount of from approximately 1 ng/ml to approximately 6000 ng/ml; optionally wherein when the bisphosphonate comprises zoledronic acid, alendronic acid or ibandronic acid, they are present in an amount of from approximately 50 ng/ml to approximately 300 ng/ml.

5. The composition according to any one of claims 1 to 4, wherein the bisphosphonate comprises, or is, zoledronic acid, optionally wherein the zoledronic acid is in an amount of from approximately 50 ng/ml to approximately 300 ng/ml.

6. The composition according to any one of claims 1 to 5, wherein when the bisphosphonate comprises:
(a) an etidronate, etidronate is present in an amount of from approximately 300 ng/ml to approximately 1500 ng/ml;
(b) a pamidronate, pamidronate is present in an amount of from approximately 0.01 nmol/ml to approximately 20 nmol/ml;
(c) a risedronate, risedronate is present in an amount of from approximately 0.01 ng/ml to approximately 50 ng/ml; and/or
(d) a clodronate, clodronate is present in an amount of from approximately 500 ng/ml to approximately 3000 ng/ml.

7. The composition according to any one of claims 1 to 6, wherein the derivative of the parathyroid hormone is selected from parathyroid hormone-related protein (PTHrP), 1-34 recombinant human parathyroid hormone (1-34 rhPTH), 1-84 recombinant human parathyroid hormone (1-84 rhPTH), H05AA03 parathyroid hormone, teriparatide, abaloparatide, and combinations of any two or more thereof.

8. The composition according to any one of claims 1 to 7, further comprising one or more additives selected from vitamin D and its derivatives of isomers thereof, hydroxyapatite and derivatives or isomers thereof, vitamin E and its derivatives of isomers thereof, selenium, zinc, magnesium, phosphate, collagen and derivatives or isomers thereof.

9. The composition according to any one of claims 1 to 8, wherein the bone void filler is selected from a calcium sulphate, a calcium phosphate, or a calcium carbonate.

10. The composition according to any one of claims 1 to 9, wherein the bone void filler comprises:
(a) a calcium sulphate, or
(b) a calcium phosphate, a calcium carbonate, an autograft bone material, an allograft, a synthetic allograft, a ceramic, a bioglass, a collagen sponge, carboxymethylcellulose or a polymethyl methacrylate (PMMA) bone cement, or a combination of any two or more thereof.

11. The composition according to any one of claims 1 to 10, wherein the composition is in a solid form or a liquid form.

12. The composition according to any one of claims 1 to 11, wherein the composition promotes new bone formation and/or suppresses bone resorption.

13. A method of manufacturing a composition according to any one of claims 1 to 12, the method comprising combining (i) the parathyroid hormone or derivative thereof; (ii) the one or more osteoclast inhibitors; and (iii) the bone void filler.

14. The composition of any one of claims 1 to 12, for use in a method of treating a bone fracture or in a bone fusion procedure.

15. The composition for use of claim 14, wherein the method comprises administering the composition to a subject,
optionally wherein the subject has delayed fracture healing in bone, and/or wherein the administering is in a fusion procedure in the skeletal system of the subject, and/or wherein the administering is to enhance bone in-filling.
